# EUROPÄISCHE PATENTSCHRIFT

(11) **EP 0 645 358 B1**
(45) Veröffentlichungstag und Bekanntmachung des Hinweises auf die Patenterteilung: **11.11.1998**
(21) Anmeldenummer: 94113561.8
(22) Anmeldetag: 31.08.1994
(51) Int. Cl.: C07C 37/58, C07C 39/07, C07C 39/08, C07B 41/00

(54) **Verfahren zur selektiven katalytischen Oxidation organischer Verbindungen**
Method of selective catalytic oxidation of organic compounds
Procédé pour l'oxidation catalytique sélective de composés organiques

(30) Priorität: 17.09.1993 DE 4331671
(43) Veröffentlichungstag der Anmeldung: 29.03.1995
(73) Patentinhaber: BAYER AG, 51368 Leverkusen (DE)
(72) Erfinder: Kricsfalussy, Zoltan, Dr., D-51375 Leverkusen (DE); Waldmann, Helmut, Dr., D-51373 Leverkusen (DE); Traenckner, Hans-Joachim, Dr., D-51375 Leverkusen (DE)

(56) Entgegenhaltungen:
- GB-A- 1 222 992
- DATABASE WPI Week 9241, Derwent Publications Ltd., London, GB; AN 92-337731 & JP-A-4 244 039 (MITSUI TOATSU)

## Beschreibung

Die vorliegende Erfindung bezieht sich auf ein verbessertes Verfahren zur selektiven katalytischen Oxidation von substituierten aromatischen Verbindungen.

Es ist bekannt, organische Verbindungen mit aktiven Sauerstoffverbindungen selektiv zu oxidieren. Als aktive Sauerstoffverbindungen kommen beispielsweise Wasserstoffperoxid, Percarbonsäuren oder Alkylhydroperoxide in Betracht. So kann man beispielsweise aus Propylen und Alkylhydroperoxiden in Gegenwart molybdänorganischer Verbindungen selektiv Propylenoxid erhalten (siehe Erdöl und Kohle, Erdgas, Petrochemie 1978/79, S. 507), aus Phenol und Wasserstoffperoxid oder Percarbonsäuren selektiv Dihydroxybenzole [siehe Chem. Ing. Techn. 62 (12), 1 041 (1990)] und aus Cyclohexanon, Ammoniak und Wasserstoffperoxid selektiv Cyclohexanonoxim (O.L. Lebedev und S.N.Kazarnovskii, Zh. Obshck Khim., 30, (1960), 1631).

Nachteilig bei diesen Verfahren ist der Einsatz von relativ schwierig herzustellenden und daher teuren Oxidationsmitteln. Bei Alkylhydroperoxiden kommt hinzu, daß der dem eingesetzten Alkylhydroperoxid entsprechende Alkohol als Koppelprodukt entsteht. Dieser muß dann einer eigenständigen Verwendung zugeführt oder nach Rückumwandlung in das Alkylhydroperoxid recyclisiert werden.

Aus DATABASE WPI Week 9241 Derwent AN 92-337 731 (= JP-A 4/244 039) ist die Oxidation von Benzol, einer unsubstituierten aromatischen Verbindung, zu Phenol bekannt, wobei mit einem Oxidationsmittel, in flüssiger Phase und mit Kupferionen und Palladium enthaltendem Katalysator gearbeitet wird. Zur Ausbeuteerhöhung können diverse Hilfsmittel und Kohlenmonoxid eingesetzt werden. Absolute Zahlenangaben zur Ausbeute liegen nicht vor.

Es wurde nun gefunden, daß man Aromaten der Formel 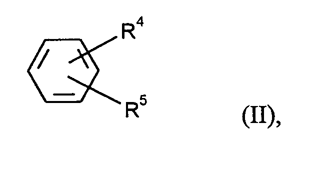 in der
R⁴ und R⁵ gleich oder verschieden sind und jeweils Wasserstoff, geradkettiges oder verzweigtes C₁-C₁₂-Alkyl, Hydroxyl, Fluor, Chlor oder Brom bedeuten, wobei jedoch R⁴ und R⁵ nicht gleichzeitig für Wasserstoff stehen, selektiv mit elementarem Sauerstoff oxidieren kann, wenn man in Gegenwart eines Katalysators, der Palladium und Kupfer enthält und in Gegenwart von Kohlenmonoxid arbeitet.

Als einzusetzende substituierte aromatische Verbindungen kommen bevorzugt Toluol, Xylol, Ethylbenzol, n-Propylbenzol, i-Butylbenzol, Phenol und Chlorbenzol infrage. Besonders bevorzugte substituierte Aromaten sind Toluol, Phenol und Chlorbenzol, insbesondere Phenol.

Beim Einsatz von Aromaten erhält man die entsprechenden kernhydroxylierten Derivate, wobei die O-H-Gruppen bevorzugt in o- oder p-Position eintreten.

Besonders bevorzugt wird aus Phenol ein Gemisch aus Brenzkatechin und Hydrochinon hergestellt.

Es ist ein wesentliches Merkmal des erfindungsgemäßen Verfahrens, daß man es in Gegenwart eines Katalysators durchführt, der Palladium und Kupfer enthält. Das Palladium kann beispielsweise in Form des Chlorides, Bromides, Acetates oder als Metall, das Kupfer beispielsweise als Kupfer(I)-Chlorid, Kupfer(II)-Chlorid, Kupfer(I)-Bromid, Kupfer(I)-Oxid, Kupfer(II)-Oxid oder als Metall vorliegen. Man kann Kupfer auch in Form einer eutektischen Salzschmelze einsetzen, beispielsweise einer Salzschmelze, die aus Kupfer(II)-Chlorid und Kaliumchlorid besteht.

Man kann die Katalysatorbestandteile, so wie sie vorliegen, dem Reaktionsgemisch bei der Durchführung des erfindungsgemäßen Verfahrens zufügen. Man kann auch einen oder beide Bestandteile auf ein Trägermaterial aufbringen, beispielsweise auf Aluminiumoxid, Siliziumdioxid, Titandioxid, Niobpentoxid, Tantaloxid, Kohlenstoff o. a. Solche Trägerkatalystoren können z.B. 1 bis 100 g/l katalytisch-aktive Substanz enthalten. Trägerkatalysatoren können z.B. angeordnet in einem Festbett oder in suspendierter Form verwendet werden.

Das Verhältnis von Palladium zu Kupfer kann in weiten Grenzen variiert werden. Berechnet als Metall kann das Äquivalent-Verhältnis von Palladium zu Kupfer beispielsweise 1:1 bis 1:1 000 betragen. Bevorzugt liegt dieses Verhältnis im Bereich 1:1 bis 1:100, besonders bevorzugt im Bereich 1:1 bis 1:10.

Das erfindungsgemäße Verfahren führt man im allgemeinen bei erhöhter Temperatur durch, beispielsweise bei 50 - 150°C. Bevorzugt sind Temperaturen im Bereich 60 bis 120°C, insbesondere im Bereich 80 bis 100°C.

Das erfindungsgemäße Verfahren kann bei den verschiedensten Drucken durchgeführt werden, beispielsweise im Bereich 1 bis 100 bar. Bevorzugt sind Drucke im Bereich 5 bis 80 bar, insbesondere im Bereich 20 bis 60 bar. Höherer Druck bedeutet im allgemeinen eine höhere Reaktionsgeschwindigkeit.

Der elementare Sauerstoff kann als technisch reines Sauerstoffgas, in Form von Luft oder im Gemisch mit inerten Gasen, z.B. im Gemisch mit Stickstoff und/oder Kohlendioxid eingesetzt werden. Sauerstoff enthaltende Gasgemische können z.B. von 2 bis 60 Vol.-% Sauerstoff enthalten.

Kohlenmonoxid kann in den zugänglichen Qualitäten eingesetzt werden.

Im allgemeinen setzt man pro Mol zu oxidierender organischer Verbindung ein Mol oder weniger Sauerstoff und ein Mol oder weniger Kohlenmonoxid ein. Es ist bevorzugt, einen Überschuß der organischen Verbindung (bezogen auf Sauerstoff) und einen Überschuß Kohlenmonoxid (bezogen auf Sauerstoff) jedoch keine höhere molare Menge Kohlenmonoxid als zu oxidierende organische Verbindung einzusetzen. Beispielsweise kann man die organische Verbindung, Kohlenmonoxid und Sauerstoff in Molverhältnissen von 1 : (1 bis 0,01) : (1 bis 0,01) einsetzen. Bevorzugt liegen die Molverhältnisse bei 1 : (0,5 bis 0,02) : (0,5 bis 0,02). Auch bei diesen relativen Einsatzmengen müssen selbstverständlich die Explosionsgrenzen des jeweiligen Systems beachtet werden, die man gegebenenfalls durch Zusätze von Inertgasen, z.B. Stickstoff und/oder Kohlendioxid, verändern kann.

In einer beispielhaften Durchführung setzt man pro Mol Phenol 0,01 bis 0,5 Mol Cu₂Cl₂ und 0,0001 bis 0,1 Mol PdCl₂ bei 80°C bis 120°C mit einem Gasgemisch von CO und Luft mit einem molaren Gehalt von 0,01 bis 0,5 Mol CO und 0,01 bis 0,5 Mol Sauerstoff bei einem Systemdruck von 30 bis 80 bar um, wobei man in 3 bis 12 Stunden ein Reaktionsgemisch mit einem Gehalt von 7 - 10 Gew.-% Brenzkatechin und Hydrochinon erhält.

Die Aufarbeitung der Reaktionsgemische erfolgt in an sich bekannter Weise. Man kann beispielsweise nach Filtration, Extraktion mit organischen Lösungsmitteln die Isolierung von Edukten und Produkten durch Destillation bewirken.

Das erfindungsgemäße Verfahren kann in den verschiedenen bekannten Reaktortypen durchgeführt werden. Man kann beispielsweise kontinuierlich in einer Blasensäule oder kontinuierlich oder diskontinuierlich in einem Rührkessel arbeiten. Als Werkstoffe für die Reaktoren eignen sich z.B. korrosionsbeständige Edelstähle, emaillierte Stähle, Glas oder Sondermetalle wie Tantal.

Das erfindungsgemäße Verfahren hat den hauptsächlichen Vorteil, daß es gestattet, substituierte aromatische Verbindungen mit dem am einfachsten und billigsten zugänglichen Oxidationsmittel selektiv zu oxidieren.

### Beispiele

### Beispiel 1

In einem mit Tantal-Einsatz versehenen Stahlbehälter, ausgestattet mit einem Begasungsrührer, Strombrecher, Thermoelement und Druckhaltung, wurden 94 g Phenol, 10 g trockenes Palladiumchlorid, 45 g Kupfer(I)-Chlorid und 4 g Wasser vorgelegt. Dazu wurden 12 l/h Kohlenmonoxid und 14 l/h Luft bei 100°C zudosiert. Der Druck wurde auf 50 bar gehalten. Nach 4 Stunden Reaktionszeit enthielt die Reaktionsmischung 1 Gew.-% Brenzkatechin und 1 Gew.-% Hydrochinon, nach 9 Stunden Reaktionszeit 4,1 Gew.-% Brenzkatechin und 2,9 Gew.-% Hydrochinon. Die Selektivität der Umsetzung von Phenol zu Brenzkatechin und Hydrochinon betrug über 95 %. Als Nebenprodukt fiel eine geringe Menge an Chlorphenolen an.

### Beispiel 2 (zum Vergleich)

Beispiel 1 wurde wiederholt, jedoch wurde kein Palladiumchlorid zugesetzt. Nach 9 Stunden konnte im Reaktionsgemisch kein Brenzkatechin und kein Hydrochinon analysiert werden.

### Beispiel 3 (zum Vergleich)

Beispiel 1 wurde wiederholt, jedoch wurde kein Kupfer(I)-Chlorid eingesetzt. Nach 9 Stunden enthielt das Reaktionsgemisch kein Brenzkatechin und nur 0,5 Gew.-% Hydrochinon.

### Beispiel 4 (zum Vergleich)

Beispiel 1 wurde wiederholt, jedoch kein Kohlenmonoxid zudosiert. Nach 9 Stunden konnten im Reaktionsgemisch nur Spuren von Brenzkatechin und Hydrochinon analysiert werden.

### Beispiel 5

Beispiel 1 wurde wiederholt, jedoch ohne Zugabe von Wasser. Nach 9 Stunden wurden im Reaktionsgemisch 0,8 Gew.-% Brenzkatechin und 0,2 Gew.-% Hydrochinon analysiert.

### Beispiel 6

In der im Beispiel 1 beschriebenen Apparatur wurden 92 g Toluol, 10 g trockenes Palladiumchlorid, 45 g Kupfer(I)-chlorid und 9 g Wasser vorgelegt. Dazu wurden 12 l/h Kohlenmonoxid und 14 l/h Luft bei 180°C zudosiert. Der Druck wurde auf 50 bar gehalten. Nach 4 Stunden Reaktionszeit enthielt die Reaktionsmischung 0,34 Gew.-% o-Kresol, 0,10 Gew.-% m-Kresol, 0,18 Gew.-% p-Kresol und 0,18 Gew.-% Benzaldehyd. Die Selektivität der Umsetzung von Toluol zu den Kresolen und Benzaldehyd betrug 90 %.

## Patentansprüche

1. Verfahren zur selektiven Oxidation von Aromaten der Formel 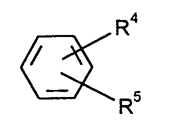 in der
R⁴ und R⁵ gleich oder verschieden sind und jeweils Wasserstoff, geradkettiges oder verzweigtes C₁-C₁₂-Alkyl, Hydroxyl, Fluor, Chlor oder Brom bedeuten, wobei jedoch R⁴ und R⁵ nicht gleichzeitig für Wasserstoff stehen, dadurch gekennzeichnet, daß man es mit elementarem Sauerstoff und in Gegenwart eines Katalysators, der Palladium und Kupfer enthält und in Gegenwart von Kohlenmonoxid durchführt.

2. Verfahren nach Anspruch 1, dadurch gekennzeichnet, daß man Toluol, Xylol, Ethylbenzol, n-Propylbenzol, 1-Butylbenzol, Phenol oder Chlorbenzol einsetzt.

## Claims

1. Process for the selective oxidation of aromatics of the formula 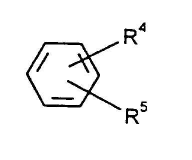 where
R⁴ and R⁵ are identical or different and are each hydrogen, straight-chain or branched C₁-C₁₂-alkyl, hydroxyl, fluorine, chlorine or bromine, but R⁴ and R⁵ are not both hydrogen, characterized in that it is carried out using elemental oxygen and in the presence of a catalyst containing palladium and copper and in the presence of carbon monoxide.

2. Process according to Claim 1, characterized in that toluene, xylene, ethylbenzene, n-propylbenzene, 1-butylbenzene, phenol or chlorobenzene is used.

## Revendications

1. Procédé pour l'oxydation sélective des composés aromatiques de formule 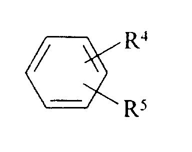 dans laquelle
R⁴ et R⁵, ayant des significations identiques ou différentes, représentent chacun l'hydrogène, un groupe alkyle à chaîne droite ou ramifiée en C₁-C₁₂, un groupe hydroxy, le fluor, le chlore ou le brome, sous réserve que R⁴ et R⁵ ne peuvent représenter tous deux l'hydrogène, caractérisé en ce que l'on opère avec de l'oxygène élémentaire, en présence d'un catalyseur qui contient du palladium et du cuivre et en présence de monoxyde de carbone.

2. Procédé selon la revendication 1, caractérisé en ce que l'on met en oeuvre le toluène, le xylène, l'éthylbenzène, le n-propylbenzène, le 1-butylbenzène, le phénol ou le chlorobenzène.
